# EUROPEAN PATENT SPECIFICATION

(11) **EP 3 727 419 B1**
(45) Date of publication and mention of the grant of the patent: **27.05.2026**
(21) Application number: 18833027.8
(22) Date of filing: 19.12.2018
(51) Int. Cl.: A61K 38/01, A61K 38/17, A61K 31/132, A61P 21/00

(54) **A COMBINATION OF SPERMIDINE AND HIGH PROTEIN COMPOSITION FOR INDUCTION OF AUTOPHAGY**
KOMBINATION VON SPERMIDIN UND HOHER PROTEINZUSAMMENSETZUNG ZUR INDUKTION VON AUTOPHAGIE
UNE COMBINAISON DE SPERMIDINE ET D'UNE COMPOSITION À HAUTE TENEUR EN PROTÉINES POUR L'INDUCTION DE L'AUTOPHAGIE

(30) Priority: 20.12.2017 US 201762608060 P
(43) Date of publication of application: 28.10.2020
(73) Proprietor: Société des Produits Nestlé S.A., 1800 Vevey (CH)
(72) Inventor: CIVILETTO, Gabriele, 1010 Lausanne (CH); CUENOUD, Bernard, 1096 CULLY (CH); FEIGE, Jerome, 1023 CRISSIER (CH); GUT, Philipp, 1201 Genève (CH)
(74) Representative: Gordon, Kirsteen Helen
(86) International application number: PCT/EP2018/085745
(87) International publication number: WO 2019/121856

(56) References cited:
- WO-A1-00/51443
- WO-A1-2011/078654
- WO-A1-2014/152610
- WO-A1-2016/078956
- WO-A1-95/13290

## Description

### BACKGROUND

The present disclosure generally relates to compositions and methods which use a combination of an autophagy inducer (spermidine) and high protein for induction of autophagy. More specifically, the present disclosure relates to administering a formulation comprising a combination of spermidine and high protein, in an amount effective to induce autophagy in muscle. The formulation can concomitantly promote protein synthesis and removal of damaged cellular materials. The recipient of administration can be a critically ill patient, for example a patient in the Intensive Care Unit (ICU), and/or an ageing patient, for example an elderly individual or a patient with sarcopenia or frailty.

Due to major advances in intensive care medicine, critically ill patients often survive acute conditions that were previously lethal. Nevertheless, mortality remains high in these patients who survive this initial phase and enter a chronic phase of critical illness. Mortality is often from non-resolving multiple organ failure, critical illness myopathy, or less severe forms of muscle weakness. Treatments have been introduced to improve muscle myopathy and weakness, such as hyperalimentation, growth hormone, or androgens, but have failed because these interventions unexpectedly increased the risk of organ failure and death. Moreover, the nutritional support to trauma and surgery patients may actually have detrimental effects.

Effective measures to provide critically ill patients with appropriate treatments and adequate nutrition remain lacking.

Moreover, age-related loss of muscle mass and function is inevitable in all individuals; however its progression largely depends on genetic and environmental factors such as physical activity and nutritional intake. Sarcopenia has been defined as the point where the age-related loss of muscle mass and function gets debilitating and impacts quality of life. In contrast, frailty is another classification of age-related physical function decline that features low muscle strength and functionality, but not muscle mass. Sarcopenia is defined clinically according to low muscle mass and function, using cutoffs which stratify the elderly population for individuals in a state of pathological mobility. Sarcopenia predicts future disability and mortality, and was assigned an official ICD-10 disease code in 2016 (Anker et al., 2016).

WO2014152610 describes biomarkers and related methods of using the biomarkers to identify subjects at risk of losing lean body mass during a prolonged period of physical inactivity. The biomarkers may be blood biomarkers and include Tissue Inhibitor of Metalloprotease-1 (TIMP-1), Tenascin C (TNC), and Apolipoprotein A2 (ApoA-2).

WO00/51443 describes a process for preserving skeletal muscle mass in a geriatric dog by feeding the dog a diet containing an effective amount of animal-based protein. Preferably, the diet includes greater than about 16 % by weight animal-based protein on a dry matter basis, and more preferably, from about 24 to 34 % by weight protein.

WO95/13290 describes a method for treating muscle disorders, such as muscular dystrophy, which demonstrate a decrease in muscle protein synthesis, an increase in protein degradation or both. The treatment comprises the administration of an effective dose of insulin-like growth factor I ("IGF-I") or genetically engineered human IGF-I (hIGF-I) to a mammalian subject.

WO2016078956 describes the treatment or prevention of conditions linked to reduced muscle mass or reduced muscle protein synthesis rate using a composition comprising complexes of whey protein micelles and pectin.

### SUMMARY

The degradation of cytoplasmic proteins is mediated by a cellular process referred to as macroautophagy, also referred to simply as autophagy. Autophagy processes are also involved in the inflammatory response and facilitate immune system destruction of bacteria. Autophagy constitutes the major lysosomal degradation pathway recycling damaged and potentially harmful cellular material such as damaged mitochondria. Notably, autophagy counteracts cell death and prolongs life span in various ageing models. As detailed in the experimental data set forth later herein, the inventors surprisingly found that autophagy inducer (spermidine) synergistically induces muscle autophagy in combination with a high protein isocaloric diet but is inactive in a low protein isocaloric diet. This synergistic induction in muscle by the combination of high protein and an autophagy inducer is in contrast to what was seen in the liver.

Accordingly, in a general embodiment, the present disclosure provides a composition comprising an effective amount of a combination of spermidine and protein, for use in the treatment or prevention of sarcopenia by inducing autophagy in skeletal muscle, wherein the protein is in an amount providing at least about 25 energy % of the composition or wherein the protein/energy ratio is greater than 6 g/100 kcal of the composition.

In an embodiment, the individual is an ageing individual.

In an embodiment, the individual has sarcopenia or is at risk of developing sarcopenia.

Although not encompassed by the wording of the claims, in an embodiment, the individual is critically ill.

Although not encompassed by the wording of the claims, in an embodiment, the individual has critical illness myopathy or is at risk of developing critical illness myopathy.

In an embodiment, at least a portion of the protein is selected from the group consisting of (i) protein from an animal source, (ii) protein from a plant source and (iii) a mixture thereof.

In an embodiment, at least a portion of the protein is selected from the group consisting of (i) milk protein, (ii) whey protein, (iii) caseinate, (iv) micellar casein, (v) pea protein, (vi) soy protein and (vii) mixtures thereof.

In an embodiment, the protein has a formulation selected from the group consisting of (i) at least 50 wt.% of the protein is casein, (ii) at least 50 wt.% of the protein is whey protein, (iii) at least 50 wt.% of the protein is pea protein and (iv) at least 50 wt.% of the protein is soy protein.

In an embodiment, at least a portion of the protein is selected from the group consisting of (i) free form amino acids, (ii) unhydrolyzed protein, (iii) partially hydrolyzed protein, (iv) extensively hydrolyzed protein, and (v) mixtures thereof. The protein can comprise one or more amino acids selected from the group consisting of histidine, isoleucine, leucine, lysine, methionine, phenylalanine, threonine, tryptophan, valine, arginine, cysteine, glutamine, glycine, proline, ornithine, serine, tyrosine, and mixtures thereof. The protein can comprise peptides having a length of 2 to 10 amino acids.

In an embodiment, the composition comprises branched chain amino acids in at least one form selected from the group consisting of (i) free form, (ii) bound to at least one additional amino acid, and (iii) mixtures thereof. The branched chain amino acids can comprise leucine in an amount effective to activate mTOR in the individual.

In an embodiment, at least a portion of the protein is 5 to 95% hydrolyzed.

In an embodiment, the protein has a formulation selected from the group consisting of (i) at least 50% of the protein has a molecular weight of 1-5 kDa, (ii) at least 50% of the protein has a molecular weight of 5-10 kDa and (iii) at least 50% of the protein has a molecular weight of 10-20 kDa.

In an embodiment, the composition comprises a carbohydrate source. The composition can have a high protein:carbohydrate ratio.

Although not encompassed by the wording of the claims, the administering uses at least one route selected from the group of oral, enteral, parenteral and intravenous injection.

The composition can be selected from the group consisting of food compositions, dietary supplements, nutritional compositions, nutraceuticals, powdered nutritional products to be reconstituted in water or milk before consumption, food additives, medicaments, drinks, and combinations thereof.

Although not encompassed by the wording of the claims, the disclosure also provides a method of making a therapeutic composition, the method comprising adding a combination of autophagy inducer (e.g., spermidine) and high protein to a base composition to form the therapeutic composition, the therapeutic composition comprising an amount of the combination per serving that is effective to induce autophagy in an individual in need thereof. The base composition can be formulated for administration by at least one route selected from the group of oral, enteral, parenteral and intravenous injection.

Although not encompassed by the wording of the claims, the disclosure provides a method comprising administering a composition comprising an amount of a combination of an autophagy inducer and high protein that concomitantly promotes protein synthesis and removal of damaged cellular materials to an individual in need thereof.

Although not encompassed by the wording of the claims, the disclosure provides a method of achieving at least one result selected from the group consisting of (i) an increased level of LC3-II protein expression or turnover, (ii) an increased level of the LC3-II / LC3-I protein ratio, (iii) a decreased level of p62 protein, (iv) a decreased level of a protein of the autophagosome, (v) an increased level of mRNA expression of an autophagy-related gene, (vi) an increased number and/or size and/or intensity of LC3 positive puncta, and (vii) degradation of LC3 and/or another autophagosome protein. The method comprises administering a therapeutically effective amount of a composition comprising a combination of an autophagy inducer and high protein to an individual in need thereof.

Although not encompassed by the wording of the claims, an advantage of one or more embodiments provided by the present disclosure is to improve the condition of critically ill animals, critically ill humans, ageing animals, or ageing humans.

Although not encompassed by the wording of the claims, another advantage of one or more embodiments provided by the present disclosure is to prevent or treat excessive catabolism, e.g., in a critically ill patient or an ageing individual.

Although not encompassed by the wording of the claims, atill another advantage of one or more embodiments provided by the present disclosure is to reduce or prevent the risk of morbidity or mortality due to excessive catabolism, e.g., in a critically ill patient or an ageing individual.

Although not encompassed by the wording of the claims, an additional advantage of the present disclosure is to reverse, treat or cure multiple organ dysfunction syndrome in a critically ill patient.

Although not encompassed by the wording of the claims, an additional advantage of one or more embodiments provided by the present disclosure is to protect an ageing individual from neurological diseases, such as mild cognitive impairment, Alzheimer disease, Parkinson's disease, Amyloid Lateral Sclerosis, Multiple Sclerosis, Huntington disease, dementia, and related neurological orphan diseases.

Although not encompassed by the wording of the claims, an additional advantage of one or more embodiments provided by the present disclosure is to protect an ageing individual from muscle dysfunction, for example, frailty, inclusion body myositis, myopathy/myolysis induced by drugs such as corticosteroids or statins, muscle wasting induced by immobilization or hospitalization.

Although not encompassed by the wording of the claims, an additional advantage of one or more embodiments provided by the present disclosure is to protect a patient suffering from a genetic disease, including but not restricted to muscular dystrophies such as Duchenne Muscular Dystrophy or Collagen VI muscular dystrophy, mitochondrial encephalomyopathies, mitochondrial myopathies, glycogen storage diseases, lysosmal storage diseases, Pompe disease.

Although not encompassed by the wording of the claims, another advantage of one or more embodiments provided by the present disclosure is a composition that can be administered parenterally or enterally, for example as an aqueous liquid composition, to a critically ill patient to induce autophagy, for example to treat multiple organ dysfunction or burn.

Although not encompassed by the wording of the claims, yet another advantage of one or more embodiments provided by the present disclosure is to decrease a length of time that a critically ill patient spends on a ventilator or to accelerate the weaning time from a ventilator.

Although not encompassed by the wording of the claims, another advantage of one or more embodiments provided by the present disclosure is to protect a critically ill patient subjected to parenteral nutrition, e.g., against multiple organ failure or muscle weakness caused by parenteral nutrient delivery, particularly unbalanced or relative nutrient overload.

Although not encompassed by the wording of the claims, an additional advantage of one or more embodiments provided by the present disclosure is to protect an ageing individual from muscle weakness.

Although not encompassed by the wording of the claims, still another advantage of one or more embodiments provided by the present disclosure is to increase the survivability of a critically ill patient or an ageing individual.

Although not encompassed by the wording of the claims, an additional advantage of one or more embodiments provided by the present disclosure is to accelerate the regain of mobility, or shorten the time of immobility, after discharge from the intensive care unit.

Although not encompassed by the wording of the claims, yet another advantage of one or more embodiments provided by the present disclosure is a beneficial effect even when a critically ill patient is already at a far-developed stage of a life threatening condition.

### BRIEF DESCRIPTION OF DRAWINGS

**FIG. 1** shows western blot images from the experimental example disclosed herein.
**FIG. 2** shows densitometric quantification of LC3-II protein amount normalized to GAPDH from the experimental example disclosed herein (A.U. : arbitrary units). The asterisks represent the significance levels calculated by ANOVA with *post hoc* Fisher test ***p < 0.001.

### DETAILED DESCRIPTION

### Definitions

Some definitions are provided hereafter. Nevertheless, definitions may be located in the "Embodiments" section below, and the above header "Definitions" does not mean that such disclosures in the "Embodiments" section are not definitions.

All percentages are by weight of the total weight of the composition unless expressed otherwise. Similarly, all ratios are by weight unless expressed otherwise. When reference is made to the pH, values correspond to pH measured at 25 °C with standard equipment. As used herein, "about," "approximately" and "substantially" are understood to refer to numbers in a range of numerals, for example the range of -10% to +10% of the referenced number, preferably -5% to +5% of the referenced number, more preferably -1% to +1% of the referenced number, most preferably -0.1% to +0.1% of the referenced number.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y." Similarly, "at least one of X or Y" should be interpreted as "X," or "Y," or "both X and Y."

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of" and "consisting of" the disclosed components. "Consisting essentially of" means that the embodiment comprises more than 50 wt.% of the identified components, preferably at least 75 wt.% of the identified components, more preferably at least 85 wt.% of the identified components, most preferably at least 95 wt.% of the identified components, for example at least 99 wt.% of the identified components.

Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

"Animal" includes, but is not limited to, mammals, which includes but is not limited to rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage, e.g., an animal capable of autophagy. As used herein, the term "patient" is understood to include an animal, for example a mammal, and preferably a human that is receiving or intended to receive treatment, as treatment is herein defined. While the terms "individual" and "patient" are often used herein to refer to a human, the present disclosure is not so limited.

Accordingly, the terms "individual" and "patient" refer to any animal, mammal or human that can benefit from the methods and compositions disclosed herein. Indeed, non-human animals undergo prolonged critical illness that mimics the human condition. These critically ill animals undergo the same metabolic, immunological and endocrine disturbances and development of organ failure and muscle wasting as the human counterpart. Moreover, animals experience the effects of ageing as well.

The term "elderly" in the context of a human means an age from birth of at least 55 years, preferably above 63 years, more preferably above 65 years, and most preferably above 70 years. The term "older adult" or "ageing individual" in the context of a human means an age from birth of at least 45 years, preferably above 50 years, more preferably above 55 years, and includes elderly individuals.

For other animals, an "older adult" or "ageing individual" has exceeded 50% of the average lifespan for its particular species and/or breed within a species. An animal is considered "elderly" if it has surpassed 66% of the average expected lifespan, preferably if it has surpassed the 75% of the average expected lifespan, more preferably if it has surpassed 80% of the average expected lifespan. An ageing cat or dog has an age from birth of at least about 5 years. An elderly cat or dog has an age from birth of at least about 7 years.

"Sarcopenia" is defined as the age-associated loss of muscle mass and functionality (including muscle strength and gait speed). As used herein, "frailty" is defined as a clinically recognizable state of increased vulnerability resulting from aging-associated decline in reserve and function across multiple physiologic systems such that the ability to cope with everyday or acute stressors is compromised. In the absence of an established quantitative standard, frailty has been operationally defined by Fried et al. as meeting three out of five phenotypic criteria indicating compromised energetics: (1) weakness (grip strength in the lowest 20% of population at baseline, adjusted for gender and body mass index), (2) poor endurance and energy (self-reported exhaustion associated with VO₂ max), (3) slowness (lowest 20% of population at baseline, based on time to walk 15 feet, adjusting for gender and standing height), (4) low physical activity (weighted score of kilocalories expended per week at baseline, lowest quintile of physical activity identified for each gender; e.g., less than 383 kcal/week for males and less than 270 kcal/week for females), and/or unintentional weight loss (10 lbs. in past year). Fried LP, Tangen CM, Walston J, et al., "Frailty in older adults: evidence for a phenotype." J. Gerontol. A. Biol. Sci. Med. Sci. 56(3):M146-M156

(2001). A pre-frail stage, in which one or two of these criteria are present, identifies a high risk of progressing to frailty.

The terms "treatment" and "treating" include any effect that results in the improvement of the condition or disorder, for example lessening, reducing, modulating, or eliminating the condition or disorder. The term does not necessarily imply that a subject is treated until total recovery. Non-limiting examples of "treating" or "treatment of" a condition or disorder include: (1) inhibiting the condition or disorder, i.e., arresting the development of the condition or disorder or its clinical symptoms and (2) relieving the condition or disorder, i.e., causing the temporary or permanent regression of the condition or disorder or its clinical symptoms. A treatment can be patient- or doctor-related.

The terms "prevention" or "preventing" mean causing the clinical symptoms of the referenced condition or disorder to not develop in an individual that may be exposed or predisposed to the condition or disorder but does not yet experience or display symptoms of the condition or disorder. The terms "condition" and "disorder" mean any disease, condition, symptom, or indication.

The relative terms "improved," "increased," "enhanced" and the like refer to the effects of the composition comprising a combination of autophagy inducer (e.g., spermidine) and high protein (disclosed herein) relative to a composition with less protein but otherwise identical.

The terms "food," "food product" and "food composition" mean a product or composition that is intended for ingestion by an individual such as a human and provides at least one nutrient to the individual. The compositions of the present disclosure, including the many embodiments described herein, can comprise, consist of, or consist essentially of the essential elements and limitations described herein, as well as any additional or optional ingredients, components, or limitations described herein or otherwise useful in a diet.

As used herein, "complete nutrition" contains sufficient types and levels of macronutrients (protein, fats and carbohydrates) and micronutrients to be sufficient to be a sole source of nutrition for the animal to which the composition is administered. Individuals can receive 100% of their nutritional requirements from such complete nutritional compositions.

As used herein, the term "critically ill patient" is an individual experiencing an acute life-threatening episode or diagnosed to be in imminent danger of such an episode. A critically ill patient is medically unstable and, when not treated, likely to die (e.g., > 50% chance of death).

Non-limiting examples of critically ill patients include a patient who has sustained or is at risk of sustaining acutely life-threatening single or multiple organ system failure due to disease or injury, a patient who is being operated upon and where complications supervene, and a patient who has a vital organ operated upon within the last week or who has been subject to major surgery within the last week.

More specific non-limiting examples of a critically ill patient include a patient who has sustained or is at risk of sustaining acutely life-threatening single or multiple organ system failure due to disease or injury and a patient who is being operated upon and where complications supervene. Additional specific non-limiting examples of a critically ill patient include a patient in need of one or more of cardiac surgery, cerebral surgery, thoracic surgery, abdominal surgery, vascular surgery, or transplantation; and a patient suffering from one or more of a neurological disease, cerebral trauma, respiratory insufficiency, abdominal peritonitis, multiple trauma, a severe burn, or critical illness polyneuropathy.

The term "Intensive Care Unit" (ICU) refers to the part of a hospital where critically ill patients are treated. The term "Intensive Care Unit" also covers a nursing home; a clinic, for example, a private clinic; or the like if the treatment activities performed there are the same or similar as those of an ICU. An "ICU patient" is encompassed by the term "critically ill patient."

The term "multiple organ dysfunction" refers to a condition resulting from infection, hypoperfusion, hypermetabolism or injury such as accident or surgery. The "multiple organ failure" of which critically ill patients die is considered a descriptive clinical syndrome defined by a dysfunction or failure of at least two vital organ systems. The vital organ systems that are uniformly and most specifically affected are the liver, the kidneys, the lungs, as well as the cardiovascular system, the nervous system and the hematological system. Non-limiting examples of multiple organ dysfunction include acute respiratory distress syndrome, heart failure, liver failure, renal failure, respiratory insufficiency, intensive care, shock, extensive burns, sepsis (e.g., systemic inflammatory response syndrome) and stroke.

The term "enterally administering" encompasses oral administration (including oral gavage administration), as well as rectal administration, although oral administration is preferred. The term "parenterally administering" refers to delivery of substances given by routes other than the digestive tract and covers administration routes such as intravenous, intra-arterial, intramuscular, intracerebroventricular, intraosseous, intradermal, intrathecal, and also intraperitoneal administration, intravesical infusion and intracavernosal injection.

Preferred parenteral administration is intravenous administration. A particular form of parenteral administration is delivery by intravenous administration of nutrition. Parenteral nutrition is "total parenteral nutrition" when no food is given by other routes. "Parenteral nutrition" is preferably a isotonic or hypertonic aqueous solution (or solid compositions to be dissolved, or liquid concentrates to be diluted to obtain an isotonic or hypertonic solution) comprising a saccharide such as glucose and further comprising one or more of lipids, amino acids, and vitamins.

### Embodiments

Although not encompassed by the wording of the claims, an aspect of the present disclosure is a method of inducing autophagy in an individual in need thereof. The method comprises administering a composition comprising a combination of autophagy inducer and high protein (e.g., about 25% of the total energy of the composition), and the composition is administered to provide an amount of the combination that is effective to induce autophagy, for example in muscle. The composition can be administered parenterally, enterally, or intravenously.

The autophagy inducer can be selected from the group consisting of spermidine, urolithin (e.g., Urolithin A, B or D), rapamycin, Torin1, valproic acid, polyphenols (e.g., resveratrol), caffeine, metformin, 5' AMP-activated protein kinase (AMPK) activators, L-type calcium channel inhibitors, and mixtures thereof. Non-limiting examples of suitable autophagy inducers are spermidine, palmitic acid, 5-aminoimidazole-4-carboxamide riboside (AICAR), verapamil, nifedipine, diltiazem, piperazine phenothiazine derivatives (e.g., trifluoperazine), ketones (e.g., beta-hydroxybutyrate, ketone salts, or ketone ester derivatives) and mixtures thereof. Non-limiting examples of suitable forms of spermidine include spermidine trihydrochloride, spermidine phosphate hexahydrate, spermidine phosphate hexahydrate, and L-arginyl-3,4-spermidine.

In an embodiment, the composition has a protein/energy ratio greater than 6 g protein/100 kcal, preferably greater than 9 g protein /100 kcal. In an embodiment, the protein is at least 24 energy % of the composition and more preferably at least 36 energy % of the composition.

As non-limiting examples, the composition can be administered in a daily dose that provides an amount of protein greater than 1.0 g protein/kg body weight/day, preferably greater than 1.2 g protein/kg body weight/day; for example up to 2.5 g protein/kg body weight/day (e.g., 1.0-2.5 g protein/kg body weight/day; 1.2-2.5 g protein/kg body weight/day; or 1.5-2.5 g protein/kg body weight/day), preferably up to 2.0 g protein/kg body weight/day (e.g., 1.0-2.0 g protein/kg body weight/day; 1.2-2.0 g protein/kg body weight/day; or 1.5-2.0 g protein/kg body weight/day), and more preferably up to 1.5 g protein/kg body weight/day (e.g., 1.0-1.5 g protein/kg body weight/day or 1.2-1.5 g protein/kg body weight/day). The daily dose of the protein can be provided by one or more servings of the composition per day.

If the composition is in liquid form, non-limiting examples of suitable high protein concentrations include 6-20 g protein/100 ml, e.g., 6-11 g protein/100 ml; 7-14 g protein/100 ml; 7-12 g protein/100 ml; 8-11 g protein/100 ml, 8-20 g protein/100 ml; 9-20 g protein/100 ml; and 11-20 g protein/100 ml.

The composition can comprise a pharmacologically effective amount of the autophagy inducer (e.g., spermidine) in a pharmaceutically suitable carrier. In aqueous liquid compositions, the autophagy inducer concentration preferably ranges from about 0.05 wt.% to about 4 wt.%, or from about 0.5 wt.% to about 2 wt.% or from about 1.0 wt.% to about 1.5 wt.% of the aqueous liquid composition.

Although not encompassed by the wording of the claims, in particular embodiments, the method is a treatment that augments the plasma spermidine level in a critically ill patient, for example to a level in the range of 50 to 6000 nmol/L plasma, preferably 100 to 6000 nmol/L plasma. The method can comprise administering daily the autophagy inducer (e.g., spermidine) in the weight range of 0.05 mg - 1 g per kg body weight, preferably 1 mg -200 mg per kg body weight, more preferably 5 mg - 150 mg per kg body weight, even more preferably 10 mg - 120 mg per kg body weight, or most preferably 40 mg - 80 mg per kg body weight.

Typically between 50 µg to 10 g of the autophagy inducer, preferably a spermidine compound, per daily serving in one or more portions is administered to a human critically ill patient.

Wheat germ is rich in spermidine. Although not encompassed by the wording of the claims,, some embodiments of the composition comprise wheat germ and/or enriched wheat germ extracts that provide at least a portion of the autophagy inducer in the composition.

The composition can induce autophagy in muscle, for example a skeletal muscle. Non-limiting examples of such muscle include one or more of the following: vastus lateralis, gastrocnemius, tibialis, soleus, extensor, digitorum longus (EDL), biceps femoris, semitendinosus, semimembranosus, gluteus maximus, extra-ocular muscles, face muscles or diaphragm.

The individual in need of induced autophagy can be an ageing individual, such as an ageing animal or an ageing human. In some embodiments, the individual in need of induced autophagy is an elderly animal or an elderly human.

The individual in need of induced autophagy can be a critically ill patient. Although not encompassed by the wording of the claims, in various embodiments, the method can treat or prevent multiple organ dysfunction in the critically ill patient, e.g., if the patient has failed or disturbed homeostasis from receiving parenteral nutrition; can protect the critically ill patient against multiple organ dysfunction; can treat or prevent development of lactic acidosis, for example lactic acidosis induced by parenteral nutrition; can treat or prevent muscle weakening in the critically ill patient; can decrease or prevent morbidity or mortality nutrition aggravated by parenteral nutrition; and/or can prevent body system collapse.

Although not encompassed by the wording of the claims, in some embodiments, the critically ill patient has at least one life threatening condition selected from the group consisting of lactic acidosis, muscle weakening, hyperglycemia, multiple organ failure, failed homeostasis, and disturbed homeostasis. In an embodiment, the critically ill patient has a non-infectious disorder. In an embodiment, the critically ill patient has multiple organ dysfunction that is not caused or associated with sepsis. Multiple organ dysfunction and muscle weakness are common in the critical care setting and can be caused or aggravated by unbalanced parenteral nutrient delivery or a parenterally delivered relative or absolute nutrient overload.

Although not encompassed by the wording of the claims, in some embodiments, the critically ill patient has at least one disorder selected from the group consisting of severe trauma, multiple trauma, high risk surgery, extensive surgery, cerebral trauma, cerebral bleeding, respiratory insufficiency, abdominal peritonitis, acute kidney injury, acute liver injury, severe burns, critical illness polyneuropathy, critical illness myopathy, and ICU-acquired muscle weakness.

Although not encompassed by the wording of the claims, in some embodiments, the critically ill patient is receiving enteral or parenteral nutrition. In some embodiments, the composition treats or prevents mitochondrial dysfunction, for example mitochondrial dysfunction induced by inadequate or unbalanced parenteral nutrition to a critically ill patient.

Although not encompassed by the wording of the claims, in another aspect of the present disclosure, a method achieves at least one result selected from the group consisting of: an increased level of LC3-II protein expression or turnover (e.g., as can be measured by western blot, mass-spectrometry, ELISA, aptamer- or nanobody-based proteomics); an increased level of the LC3-II / LC3-I protein ratio (e.g., as can be measured by any of the methods above); a decreased level of p62 protein (e.g., as can be measured by the aforementioned methods); a decreased level of a protein of the autophagosome, for example but not limited to Atg5, Beclin-1, Atg7, Atg12; an increased level of mRNA expression of autophagy related genes, for example but not limited to MAP1LC3, GABARAP, Atg5, Beclin-1, Atg7, Atg12; and increased number and/or size and/or intensity of LC3 positive puncta (as can be assessed by immunofluorescence or by tagging LC3 to a fluorescent reporter protein like GFP or by flow cytometry); degradation of LC3 and/or another autophagosome protein (as can be measured by assessing its lysosomal degradation assessed by microscopy or flow cytometry, for example by fusing the protein to a pH sensitive fluorescent reporter that will change color when reaching the lysosome; or can be measured by comparing the fluorescent intensity of the WT protein to a mutated protein which cannot be inserted in autophagosomes, for example, the LC3ΔG mutant which cannot be lipidated and inserted in the autophagosome). The method comprises administering a therapeutically effective amount of a composition comprising a combination of an autophagy inducer and high protein to an individual in need thereof.

The term "protein" as used herein includes free form amino acids, molecules between 2 and 20 amino acids (referenced herein as "peptides"), and also includes longer chains of amino acids as well. Small peptides, i.e., chains of 2 to 10 amino acids, are suitable for the composition alone or in combination with other proteins. The "free form" of an amino acid is the monomeric form of the amino acid. Suitable amino acids include both natural and non-natural amino acids. The composition can comprise a mixture of one or more types of protein, for example one or more (i) peptides, (ii) longer chains of amino acids, or (iii) free form amino acids; and the mixture is preferably formulated to achieve a desired amino acid profile/content.

At least a portion of the protein can be from animal or plant origin, for example dairy protein such as one or more of milk protein, e.g., milk protein concentrate or milk protein isolate; caseinates or casein, e.g., micellar casein concentrate or micellar casein isolate; or whey protein, e.g., whey protein concentrate or whey protein isolate. Additionally or alternatively, at least a portion of the protein can be plant protein such as one or more of soy protein or pea protein.

Mixtures of these proteins are also suitable, for example mixtures in which casein is the majority of the protein but not the entirety, mixtures in which whey protein is the majority of the protein but not the entirety, mixtures in which pea protein is the majority of the protein but not the entirety, and mixtures in which soy protein is the majority of the protein but not the entirety. In an embodiment, at least 10 wt.% of the protein is whey protein, preferably at least 20 wt.%, and more preferably at least 30 wt.%. In an embodiment, at least 10 wt.% of the protein is casein, preferably at least 20 wt.%, and more preferably at least 30 wt.%. In an embodiment, at least 10 wt.% of the protein is plant protein, preferably at least 20 wt.%, more preferably at least 30 wt.%.

Whey protein may be any whey protein, for example selected from the group consisting of whey protein concentrates, whey protein isolates, whey protein micelles, whey protein hydrolysates, acid whey, sweet whey, modified sweet whey (sweet whey from which the caseino-glycomacropeptide has been removed), a fraction of whey protein, and any combination thereof.

Casein may be obtained from any mammal but is preferably obtained from cow milk and preferably as micellar casein.

The protein may be unhydrolyzed, partially hydrolyzed (i.e., peptides of molecular weight 3 kDa to 10 kDa with an average molecular weight less than 5 kDa) or extensively hydrolyzed (i.e., peptides of which 90% have a molecular weight less than 3 kDa), for example in a range of 5% to 95% hydrolyzed. In some embodiments, the peptide profile of hydrolyzed protein can be within a range of distinct molecular weights. For example, the majority of peptides (>50 molar percent or > 50 wt.%) can have a molecular weight within 1-5 kDa, or 5-10 kDa, or 10-20 kDa.

The protein can comprise essential amino acids and/or conditionally essential amino acids, e.g., such amino acids that may be insufficiently delivered in a caloric restriction regimen. For example, the protein can comprise one or more essential amino acids selected from the group consisting of histidine, isoleucine, leucine, lysine, methionine, phenylalanine, threonine, tryptophan, and valine; and each of these amino acids (if present) may be administered in the composition in a daily dose from about 0.0476 to about 47.6 mg amino acid/kg bw. Notably, lower intake of methionine leads to lower levels of protein translation and ultimately muscle synthesis. The protein can comprise one or more conditionally essential amino acids (e.g., amino acids conditionally essential in illness or stress) selected from the group consisting of arginine, cysteine, glutamine, glycine, proline, ornithine, serine and tyrosine; and each of these amino acids (if present) may be administered in the composition in a daily dose from about 0.0476 to about 47.6 mg amino acid/kg bw.

The composition can comprise one or more branched chain amino acids (BCAAs). For example, the composition can comprise leucine, isoleucine and/or valine, in free form and/or bound as peptides and/or proteins such as dairy, animal or plant proteins. A daily dose of the branched chain amino acids can include one or more of 0.3 5-142.85 mg/kg bw Leucine, preferably 0.175-71.425 mg/kg bw Leucine; 0.175-71.425 mg/kg bw Isoleucine; and 0.175-71.425mg/kg bw Valine. The daily dose of the one or more branched chain amino acids can be provided by one or more servings of the composition per day.

Whey protein is rich in BCAAs. Therefore, some embodiments of the composition comprise whey protein that provides at least a portion of the BCAAs in the composition.

In an embodiment, the composition includes a source of carbohydrates. Any suitable carbohydrate may be used in the composition including, but not limited to, starch (e.g., modified starch, amylose starch, tapioca starch, corn starch), sucrose, lactose, glucose, fructose, corn syrup solids, maltodextrin, xylitol, sorbitol or combinations thereof.

The source of carbohydrates is preferably not greater than 50 energy % of the composition, more preferably not greater than 36 energy % of the composition, and most preferably not greater than 30 energy % of the composition. The composition can have a high protein:carbohydrate energy ratio, for example greater than 0.66, preferably greater than 0.9 and more preferably greater than 1.2.

Although not encompassed by the wording of the claims, in an embodiment, the composition includes a source of fat. The source of fat may include any suitable fat or fat mixture. Non-limiting examples of suitable fat sources include vegetable fat, such as olive oil, corn oil, sunflower oil, high-oleic sunflower, rapeseed oil, canola oil, hazelnut oil, soy oil, palm oil, coconut oil, blackcurrant seed oil, borage oil, lecithins, and the like, animal fats such as milk fat; or combinations thereof.

The composition comprising a combination of autophagy inducer (e.g., spermidine) and high protein can be administered to an individual such as a human, e.g., an ageing individual or a critically ill individual, in a therapeutically effective dose. The therapeutically effective dose can be determined by the person skilled in the art and will depend on a number of factors known to those of skill in the art, such as the severity of the condition and the weight and general state of the individual.

The composition is preferably administered to the individual at least two days per week, more preferably at least three days per week, most preferably all seven days of the week; for at least one week, at least one month, at least two months, at least three months, at least six months, or even longer. Although not encompassed by the wording of the claims, in some embodiments, the composition is administered to the individual consecutively for a number of days, for example at least until a therapeutic effect is achieved. In an embodiment, the composition can be administered to the individual daily for at least 30, 60 or 90 consecutive days.

The above examples of administration do not require continuous daily administration with no interruptions. Instead, there may be some short breaks in the administration, such as a break of two to four days during the period of administration. The ideal duration of the administration of the composition can be determined by those of skill in the art.

In a preferred embodiment, the composition is administered to the individual orally or enterally (e.g. tube feeding). For example, the composition can be administered to the individual as a beverage, a capsule, a tablet, a powder or a suspension.

The composition can be any kind of composition that is suitable for human and/or animal consumption. For example, the composition may be selected from the group consisting of food compositions, dietary supplements, nutritional compositions, nutraceuticals, powdered nutritional products to be reconstituted in water or milk before consumption, food additives, medicaments, beverages and drinks. In an embodiment, the composition is an oral nutritional supplement (ONS), a complete nutritional formula, a pharmaceutical, a medical or a food product. In a preferred embodiment, the composition is administered to the individual as a beverage. The composition may be stored in a sachet as a powder and then suspended in a liquid such as water for use.

In some instances where oral or enteral administration is not possible or not advised, the composition may also be administered parenterally.

In some embodiments, the composition is administered to the individual in a single dosage form, i.e. all compounds are present in one product to be given to an individual in combination with a meal. In other embodiments, the composition is co-administered in separate dosage forms, for example at least one component separately from one or more of the other components of the composition.

### EXAMPLES

The following non-limiting examples present scientific data developing and supporting the concept of administering a composition comprising a combination of autophagy inducer (e.g., spermidine) and high protein to induce autophagy in an individual in need thereof.

### Material and methods

### Mice

10-15 week-old C57bl6/J mice were fed during ad libitum for 4 weeks with one of: a standard diet, a diet rich in proteins and poor in carbohydrates (high protein isocaloric), or a diet poor in proteins and rich in carbohydrates (low protein isocaloric). Each group was treated or not with 3mM of autophagy inducer (e.g., spermidine) in the drinking water. Standard diet was composed of 16% fats (Soybean oil), 20% proteins (casein) and 64% carbohydrates (40% cornstarch, 14% dextrinized cornstarch, 10% sucrose). Low protein diet was composed of 16% fats (Soybean oil), 5% proteins (casein) and 79% carbohydrates (62% cornstarch, 10% dextrinized cornstarch, 7% sucrose). High protein / low carb diet was composed of 16% fats (Soybean oil), 60% proteins (casein) and 24% carbohydrates (7% cornstarch, 10% dextrinized cornstarch, 7% sucrose). These amounts are percentages of total energy of the feed. Mice were sacrificed by isofluoran inhalation followed by exsanguination. Quadricep muscles were collected and frozen in liquid nitrogen.

### Western Blots

Total protein lysates were extracted from 30-50 mg of tissues homogenized in 20 ml/g of RIPA buffer (150 mM sodium chloride, 50 mM Tris pH: 8, 1% Triton X-100, 0.5% deoxycolate, 0.1% SDS, protease inhibitors cocktail) with a tissue dissociator (gentleMACS Miltenyi Biotec). Protein concentration was determined by BCA assay, and samples were prepared adding 4X LDS sample buffer (Invitrogen). 20 µg of proteins were separated by SDS-PAGE in 4-12% gradient gels and transferred to PVDF membranes using dry iBLOT system (Invitrogen). Membranes were incubated with LC3 (Novus Biologicals 2220) and GAPDH (Cell Signaling 2118) antibodies and detected with ECL substrates (Pierce). Protein quantification was performed by densitometric analysis of images using ImageJ software.

### Results

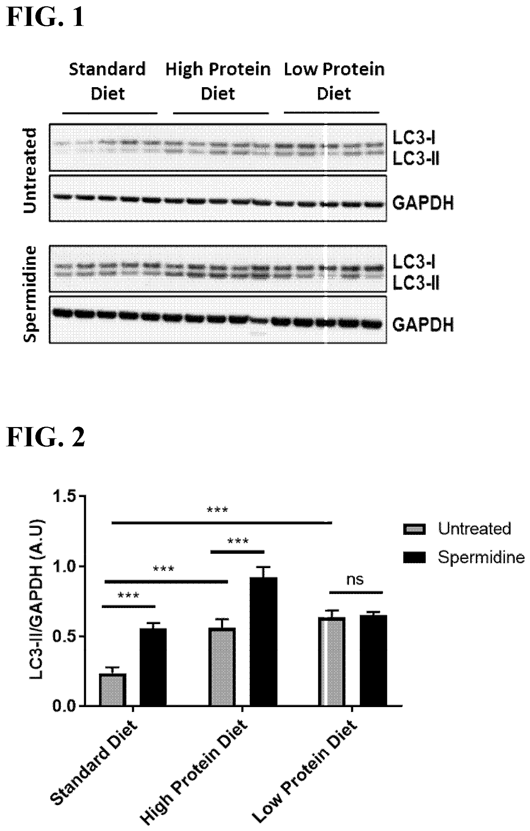

**FIGS. 1** and **2** show the results from young mice fed with one of a standard diet, a diet rich in proteins / poor in carbohydrates (high protein diet) or a diet poor in proteins / rich in carbohydrates (low protein diet), and treated or not with 3mM of Autophagy inducer (e.g., spermidine) in the drinking water to achieve an exposure of 105+/-35 mg/kg body weight/day. LC3-I and LC3-II protein amounts were measured in skeletal muscle (quadriceps) by western blot and normalized to GAPDH. Densitometric quantification of LC3-II protein amount normalized to GAPDH (A.U. : arbitrary units). The asterisks represent the significance levels calculate by ANOVA with *post hoc* Fisher test ***p < 0.001. These results surprisingly demonstrate that autophagy inducer (e.g., spermidine) synergistically induces muscle autophagy in combination with a high-protein isocaloric diet and is inactive in a low protein isocaloric diet.

## Claims

1. A composition comprising an effective amount of a combination of spermidine and protein for use in the treatment or prevention of sarcopenia by inducing autophagy in skeletal muscle, wherein the protein is in an amount providing at least about 25 energy % of the composition or wherein the protein/energy ratio is greater than 6 g/100 kcal of the composition.

2. The composition for use of claim 1 wherein at least a portion of the protein is selected from the group consisting of (i) protein from an animal source, (ii) protein from a plant source and (iii) a mixture thereof.

3. The composition for use of claim 1 wherein at least a portion of the protein is selected from the group consisting of (i) milk protein, (ii) whey protein, (iii) caseinate, (iv) micellar casein, (v) pea protein, (vi) soy protein and (vii) mixtures thereof.

4. The composition for use of claim 3 wherein the protein is micellar casein.

5. The composition for use of claim 1 wherein the protein has a formulation selected from the group consisting of (i) at least 50 wt.% of the protein is casein, (ii) at least 50 wt.% of the protein is whey protein, (iii) at least 50 wt.% of the protein is pea protein and (iv) at least 50 wt.% of the protein is soy protein.

6. The composition for use of claim 1 wherein at least a portion of the protein is selected from the group consisting of (i) free form amino acids, (ii) unhydrolyzed protein, (iii) partially hydrolyzed protein, (iv) extensively hydrolyzed protein, and (v) mixtures thereof.

7. The composition for use of claim 6 wherein the protein comprises peptides having a length of 2 to 10 amino acids.

8. The composition for use of claim 1 wherein the protein comprises branched chain amino acids in at least one form selected from the group consisting of (i) free form, (ii) bound to at least one additional amino acid, and (iii) mixtures thereof.

9. The composition for use of claim 8 wherein the branched chain amino acids comprise leucine in an amount effective to activate mTOR in the individual.

10. The composition for use of claim 1 wherein at least a portion of the protein is 5 to 95% hydrolyzed.

11. The composition for use of claim 1 wherein the protein has a formulation selected from the group consisting of (i) at least 50% of the protein has a molecular weight of 1-5 kDa, (ii) at least 50% of the protein has a molecular weight of 5-10 kDa and (iii) at least 50% of the protein has a molecular weight of 10-20 kDa.

12. The composition for use of claim 1 wherein the composition comprises a carbohydrate source.

13. The composition for use of claim 12 wherein the composition has a high protein: carbohydrate ratio.

14. The composition for use according to any one of claims 1 to 16 wherein the composition is selected from the group consisting of food compositions, dietary supplements, nutritional compositions, nutraceuticals, powdered nutritional products to be reconstituted in water or milk before consumption, food additives, medicaments, drinks, and combinations thereof.

15. The composition for use according to claim 1, wherein the protein is in an amount providing at least about 36 energy % of the composition.

## Patentansprüche

1. Zusammensetzung, umfassend eine wirksame Menge einer Kombination aus Spermidin und Protein zur Verwendung bei der Behandlung oder Vorbeugung von Sarkopenie durch ein Induzieren einer Autophagie in einem Skelettmuskel, wobei das Protein in einer Menge vorliegt, die mindestens etwa 25 Energie-% der Zusammensetzung bereitstellt, oder wobei das Protein/Energie-Verhältnis größer als 6 g/100 kcal der Zusammensetzung ist.

2. Zusammensetzung zur Verwendung nach Anspruch 1, wobei mindestens ein Anteil des Proteins aus der Gruppe ausgewählt ist, bestehend aus (i) Protein aus einer tierischen Quelle, (ii) Protein aus einer pflanzlichen Quelle und (iii) einer Mischung davon.

3. Zusammensetzung zur Verwendung nach Anspruch 1, wobei mindestens ein Anteil des Proteins aus der Gruppe ausgewählt ist, bestehend aus (i) Milchprotein, (ii) Molkenprotein, (iii) Caseinat, (iv) mizellarem Casein, (v) Erbsenprotein, (vi) Sojaprotein und (vii) Mischungen davon.

4. Zusammensetzung zur Verwendung nach Anspruch 3, wobei das Protein mizellares Casein ist.

5. Zusammensetzung zur Verwendung nach Anspruch 1, wobei das Protein eine Formulierung aufweist, die aus der Gruppe ausgewählt ist, bestehend aus (i) mindestens 50 Gew.-% des Proteins ist Casein, (ii) mindestens 50 Gew.-% des Proteins ist Molkenprotein, (iii) mindestens 50 Gew.-% des Proteins ist Erbsenprotein und (iv) mindestens 50 Gew.-% des Proteins ist Sojaprotein.

6. Zusammensetzung zur Verwendung nach Anspruch 1, wobei mindestens ein Anteil des Proteins aus der Gruppe ausgewählt ist, bestehend aus (i) Aminosäuren in freier Form, (ii) nicht hydrolysiertem Protein, (iii) teilweise hydrolysiertem Protein, (iv) ausführlich hydrolysiertem Protein und (v) Mischungen davon.

7. Zusammensetzung zur Verwendung nach Anspruch 6, wobei das Protein Peptide umfasst, die eine Länge von 2 bis 10 Aminosäuren aufweisen.

8. Zusammensetzung zur Verwendung nach Anspruch 1, wobei das Protein verzweigtkettige Aminosäuren in mindestens einer Form umfasst, die aus der Gruppe ausgewählt ist, bestehend aus (i) freier Form, (ii) an mindestens eine zusätzliche Aminosäure gebunden und (iii) Mischungen davon.

9. Zusammensetzung zur Verwendung nach Anspruch 8, wobei die verzweigtkettigen Aminosäuren Leucin in einer Menge umfassen, die wirksam ist, um mTOR in dem Individuum zu aktivieren.

10. Zusammensetzung zur Verwendung nach Anspruch 1, wobei mindestens ein Anteil des Proteins zu 5 bis 95 % hydrolysiert ist.

11. Zusammensetzung zur Verwendung nach Anspruch 1, wobei das Protein eine Formulierung aufweist, die aus der Gruppe ausgewählt ist, bestehend aus (i) mindestens 50 % des Proteins weist ein Molekulargewicht von 1-5 kDa auf, (ii) mindestens 50 % des Proteins weist ein Molekulargewicht von 5-10 kDa auf und (iii) mindestens 50 % des Proteins weist ein Molekulargewicht von 10-20 kDa auf.

12. Zusammensetzung zur Verwendung nach Anspruch 1, wobei die Zusammensetzung eine Kohlenhydratquelle umfasst.

13. Zusammensetzung zur Verwendung nach Anspruch 12, wobei die Zusammensetzung ein hohes Protein : Kohlenhydrat-Verhältnis aufweist.

14. Zusammensetzung zur Verwendung nach einem der Ansprüche 1 bis 16, wobei die Zusammensetzung aus der Gruppe ausgewählt ist, bestehend aus Nahrungsmittelzusammensetzungen, Nahrungsergänzungsmitteln, Nährstoffzusammensetzungen, Nutrazeutika, pulverisierten Nährstoffprodukten, die vor einem Verzehr in Wasser oder Milch aufzulösen sind, Nahrungsmittelzusätzen, Medikamenten, Getränken und Kombinationen davon.

15. Zusammensetzung zur Verwendung nach Anspruch 1, wobei das Protein in einer Menge vorliegt, die mindestens etwa 36 Energie-% der Zusammensetzung bereitstellt.

## Revendications

1. Composition comprenant une quantité efficace d'une combinaison de spermidine et de protéine pour une utilisation dans le traitement ou la prévention de la sarcopénie par induction de l'autophagie dans un muscle squelettique, dans laquelle la protéine est en une quantité fournissant au moins environ 25 % de l'énergie de la composition ou dans laquelle le rapport protéine/énergie est supérieur à 6 g/100 kcal de la composition.

2. Composition pour une utilisation selon la revendication 1, dans laquelle au moins une partie de la protéine est choisie dans le groupe constitué de (i) une protéine d'origine animale, (ii) une protéine d'origine végétale et (iii) un mélange de celles-ci.

3. Composition pour une utilisation selon la revendication 1, dans laquelle au moins une partie de la protéine est choisie dans le groupe constitué de (i) une protéine de lait, (ii) une protéine de lactosérum, (iii) un caséinate, (iv) une caséine micellaire, (v) une protéine de pois, (vi) une protéine de soja et (vii) de mélanges de ceux-ci.

4. Composition pour une utilisation selon la revendication 3, dans laquelle la protéine est la caséine micellaire.

5. Composition pour une utilisation selon la revendication 1, dans laquelle la protéine a une formulation choisie dans le groupe constitué de (i) au moins 50 % en poids de la protéine est de la caséine, (ii) au moins 50 % en poids de la protéine est une protéine de lactosérum, (iii) au moins 50 % en poids de la protéine est une protéine de pois et (iv) au moins 50 % en poids de la protéine est une protéine de soja.

6. Composition pour une utilisation selon la revendication 1, dans laquelle au moins une partie de la protéine est choisie dans le groupe constitué de (i) acides aminés sous forme libre, (ii) une protéine non hydrolysée, (iii) une protéine partiellement hydrolysée, (iv) une protéine extensivement hydrolysée, et (v) de mélanges de ceux-ci.

7. Composition pour une utilisation selon la revendication 6, dans laquelle la protéine comprend des peptides ayant une longueur de 2 à 10 acides aminés.

8. Composition pour une utilisation selon la revendication 1, dans laquelle la protéine comprend des acides aminés à chaîne ramifiée sous au moins une forme choisie dans le groupe constitué de (i) une forme libre, (ii) liée à au moins un acide aminé supplémentaire, et (iii) de mélanges de celles-ci.

9. Composition pour une utilisation selon la revendication 8, dans laquelle les acides aminés à chaîne ramifiée comprennent de la leucine en une quantité efficace pour activer mTOR chez l'individu.

10. Composition pour une utilisation selon la revendication 1, dans laquelle au moins une partie de la protéine est hydrolysée de 5 à 95 %.

11. Composition pour une utilisation selon la revendication 1, dans laquelle la protéine a une formulation choisie dans le groupe constitué de (i) au moins 50 % de la protéine a un masse moléculaire de 1 à 5 kDa, (ii) au moins 50 % de la protéine a une masse moléculaire de 5 à 10 kDa et (iii) au moins 50 % de la protéine a une masse moléculaire de 10 à 20 kDa.

12. Composition pour une utilisation selon la revendication 1, dans laquelle la composition comprend une source de glucides.

13. Composition pour une utilisation selon la revendication 12, dans laquelle la composition a un rapport protéine: glucides élevé.

14. Composition pour une utilisation selon l'une quelconque des revendications 1 à 16, dans laquelle la composition est choisie dans le groupe constitué de compositions alimentaires, compléments alimentaires, compositions nutritionnelles, produits nutraceutiques, produits nutritionnels en poudre destinés à être reconstitués dans de l'eau ou du lait avant consommation, additifs alimentaires, médicaments, boissons et de combinaisons de ceux-ci.

15. Composition pour une utilisation selon la revendication 1, dans laquelle la protéine est en une quantité fournissant au moins environ 36 % de l'énergie de la composition.
